# EUROPEAN PATENT SPECIFICATION

(11) **EP 2 097 015 B1**
(45) Date of publication and mention of the grant of the patent: **27.02.2013**
(21) Application number: 07864648.6
(22) Date of filing: 20.11.2007
(51) Int. Cl.: A61B 17/17, A61B 17/80

(54) **VARIABLE ANGLE DRILL GUIDE**
BOHRFÜHRUNG MIT VARIABLEM WINKEL
GUIDE-FORET À ANGLE VARIABLE

(30) Priority: 21.11.2006 US 866665 P
(43) Date of publication of application: 09.09.2009
(60) Divisional application: 12006811.9
(73) Proprietor: Smith & Nephew, Inc., Memphis, TN 38116 (US)
(72) Inventor: CHREENE, David E., Hernando, Mississippi 38632 (US); BAKER, Charles R., Lakeland, TN 38002 (US); STEWART, William W., Memphis, TN 38104 (US); ZAHLRY, Daniel C., Germantown, TN 38139 (US)
(74) Representative: Connors, Martin L.H.
(86) International application number: PCT/US2007/085210
(87) International publication number: WO 2008/064211

(56) References cited:
- WO-A-02/080791
- DE-U1- 20 019 026
- US-A- 5 669 915
- US-A1- 2003 083 667

## Description

### BACKGROUND OF THE INVENTION

### FIELD OF THE INVENTION

The present invention relates generally to surgical instruments and, more particularly, to a variable angle drill guide.

### RELATED ART

Presently, variable angle drill guides for use with bone plating in the medical field require the user to drill through multiple sleeves or require multiple complex components of assembly to pivot for off-axis drilling. It would be desirable to provide the ability to rotate 360 degrees relative to the longitudinal axis of the drill guide while drilling on or off-axis. It also would be desirable to angle the drill bit through a single sleeve or guide feature while the drill guide itself is oriented transverse to the bone plate surface, which in turn ensures that a fastener is located in the center of the hole or other plate opening while drilling at any angle.

International Patent Application No. PCT/US2005/012116 discloses a surgical drill guide for use with a bone plate having fastener holes oriented at predetermined angles with respect to the plate. The surgical drill guide has at least one alignment drill guide barrel that is aligned with the respective fastener holes in the bone plate for drilling holes at the desired range of angles permitted by the plate hole. However, this reference does not disclose a mechanism that positively engages the bone plate nor does it provide a limit on the angularity of the drill bit.

International Patent Application No. PCT/CH01/00221 discloses a surgical drill guide for demountable attachment to a slotted bone plate. The drill guide assembly includes one or more alignment drill tubes that are remotely aligned with corresponding fastener holes in the bone plate and an expandable bushing that is configured and dimensioned to engage a slot in the bone plate. A variable angle block permits angulation of the alignment drill tubes about a central axis of the surgical drill guide assembly. The surgical drill tubes are releasably lockable at a surgeon-selected angle. However, this reference fails to disclose a drill guide proximate to the bone plate. Document US2003083667 A discloses another drill guide assembly with variable drilling angle.

There remains a need in the art for a variable angle drill guide that provides support for the drill bit proximate a bone plate and limits the maximum angle of the drill bit relative to the bone plate hole.

### SUMMARY OF THE INVENTION

A drill guide assembly (10;1100;1200) adapted for removable connection to a bone plate (200;1150;1250) and for guiding a drill bit (300) through at least one hole (202) of the bone plate, the at least one hole having a central axis, the drill guide assembly comprising:
a. a guide sleeve (80;1110) having a proximal end portion (86) and a distal end portion (88), a central axis and a length, the guide sleeve also having a bore (84) and a passage (90) wherein the passage (90) extend at least a portion of the length of the guide sleeve, the guide sleeve being coaxial with the central axis of the at least one hole and adapted to swivel about the central axis of the at least one hole while the distal end portion maintains contact with the bone plate; and
b. a guide collar (30) operatively connected to the proximal end portion, the guide collar having an outrigger (40), the outrigger having a slot (42) which is adapted to receive the drill bit and which is aligned with the passage (90), the slot (42) allowing an operator to select an angle for placement of the drill bit with respect to the central axis of the guide sleeve, and wherein the length of the slot (42) is dimensioned according to the desired maximum angle of the drill.

In some embodiments, the outrigger further comprises a flanged bobbin.

In some embodiments, the outrigger further comprises a plurality of scallops.

In some embodiments, the drill guide assembly further comprises at least one ball plunger.

In some embodiments, the guide collar includes a base and a body portion, and the outrigger extends generally from the body portion.

In some embodiment, the guide sleeve includes a sleeve body and a passage.

In some embodiments, the guide sleeve further comprises at least one marker groove.

In some embodiments, the outrigger includes one or more markings to indicate an angle of the drill bit.

In some embodiments, the drill guide assembly further comprises a drill guide handle, the drill guide handle having a grip portion and an engagement portion.

In some embodiments, the drill guide handle further comprises a pivot portion.

In some embodiments, the distal end portion of the guide sleeve comprises a spherical tip.

In some embodiments, the drill guide assembly further comprises a bushing and a distal guide tip.

In some embodiments, the drill guide assembly further comprises a limiter. The limiter may or may not threadingly engage the guide sleeve.

In some embodiments, the distal end portion of the guide sleeve comprises a drill guide tip. The drill guide tip may include an anti-rotation feature. The anti-rotation feature may be a locating boss, a locating member, or comprise at least one thread adapted to mate with at least one thread of the at least one bone plate hole.

In some embodiments, the guide sleeve further comprises at least one tang and the drill guide tip further comprises an under-cut groove adapted to receive the at least one tang.

In some embodiments, the drill guide tip further comprises a cone.

There is further provided a drill guide assembly adapted for removable connection to a bone plate and for guiding a drill bit through at least one hole of the bone plate, the at least one hole having a central axis, the drill guide assembly comprising: a guide sleeve assembly having a proximal end portion and a distal end portion, the guide sleeve assembly coaxial with the central axis and adapted to swivel about the central axis while the distal end portion maintains contact with the bone plate, the guide sleeve assembly comprising an outer guide sleeve and an inner guide sleeve pivotally connected to the outer guide sleeve, the inner guide sleeve having at least one opening adapted to receive the drill hit, and the at least one opening allowing an operator to select an angle for placement of the drill bit and adapted to limit the maximum angle selectable by the operator.

There is also provided a drill guide assembly for guiding a drill bit through at least one hole of a bone plate, the drill guide assembly comprising: a guide collar, the guide collar having an outrigger, and the outrigger having a slot adapted to receive the drill bit; a guide sleeve connected to the guide collar, the guide sleeve having a passage and a length, the passage extending at least a portion of the length of the guide sleeve; and a drill guide tip connected to the guide sleeve, the drill guide tip adapted for removable connection to the bone plate, and the drill guide tip having a cone, wherein a movement of the drill is limited by the slot, the passage, and the cone.

There is also provided a drill guide assembly for guiding a drill bit through at least one hole of a bone plate, the drill guide assembly comprising: a guide collar, the guide collar having a basle, a body portion, and an outrigger, the body portion extending from the base in a transverse direction, the outrigger extending from body portion in a generally arcuate direction, and the outrigger having a slot adapted to receive the drill bit and limit the maximum angle of the drill bit; a guide sleeve connected to the guide collar, the guide sleeve having a sleeve body, a bore, a passage, a plurality of tangs, and a length, the bore extending the length of the sleeve body, and the passage extending at least a portion of the length, and a drill guide tip connected to the guide sleeve, the drill guide tip having an under-cut groove to receive the plurality of tangs, a cone, and a locating boss, the locating boss adapted for removable connection to the bone plate, and wherein a movement of the drill is limited by the slot, the passage, and the cone.

There is provided a variable angle drill guide assembly for use in bone plating applications. The variable angle drill guide assembly includes a guide collar, a guide sleeve, and a guide tip. The guide tip interfaces with a bone plate in a transverse manner and positions the variable angle drill guide in the center of a bone plate hole. The variable angle drill guide is applicable for use with either locking or non-locking holes. The variable angle drill guide allows the user to drill on or off-axis at any angle from about zero to about thirty degrees, as well as in any direction of 360 degrees of rotation about the longitudinal axis of the drill guide.

The variable angle drill guide assembly offers more opportunities for fracture fixation in comparison to prior devices. The rotational freedom and angular freedom increases the user's opportunities for bone fracture fucation. In particular, the rotational freedom is not limited to predetermined hole locations of the bone plate.

The variable angle drill guide assembly provides an advantage over prior devices because it is simpler to use and reduces the overall number of instruments required for the surgical technique. The variable angle drill guide assembly eliminates the need for multiple barrels or guide sleeves, which also reduces the time required to complete the surgical technique.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

The accompanying drawings, which are incorporated in and form a part of the specification, illustrate the embodiments of the present invention and together with the written description serve to explain the principles, characteristics, and features of the invention. In the drawings:

FIG. 1 is an exploded, front perspective view of a variable angle drill guide assembly, handle and bone plate;

FIG. 2 is an exploded, front perspective view of a guide collar;

FIG. 3 is a front perspective view of a guide sleeve;

FIG. 4 is a partial sectional front view of a guide tip;

FIG. 5 is a bottom view of the guide tip in a first embodiment;

FIG. 6 is a bottom view of the guide tip in a second embodiment;

FIG. 7 is a bottom view of the guide tip in a third embodiment;

FIG. 8 is a front perspective view of the variable angle drill guide assembly, a bone, and a bone plate;

FIG. 9 is a sectional front view of the variable angle drill guide assembly shown in FIG. 8;

FIG. 10 is a front perspective view of an alternative embodiment of the handle;

FIG. 11 is a sectional side view of the handle shown in FIG. 10;

FIG. 12 is a sectional side view of the outrigger in a second embodiment;

FIG. 13 is a partial top view of the outrigger in a third embodiment;

FIG. 14 is a front perspective view of an alternative embodiment of the drill guide assembly; and

FIG. 15 is a front view of an alternative method of limiting the relationship between the bone plate and the drill guide assembly.

### DETAILED DESCRIPTION OF THE EMBODIMENT

The following description of the preferred embodiment(s) is merely exemplary in nature and is in no way intended to limit the invention, its application, or uses.

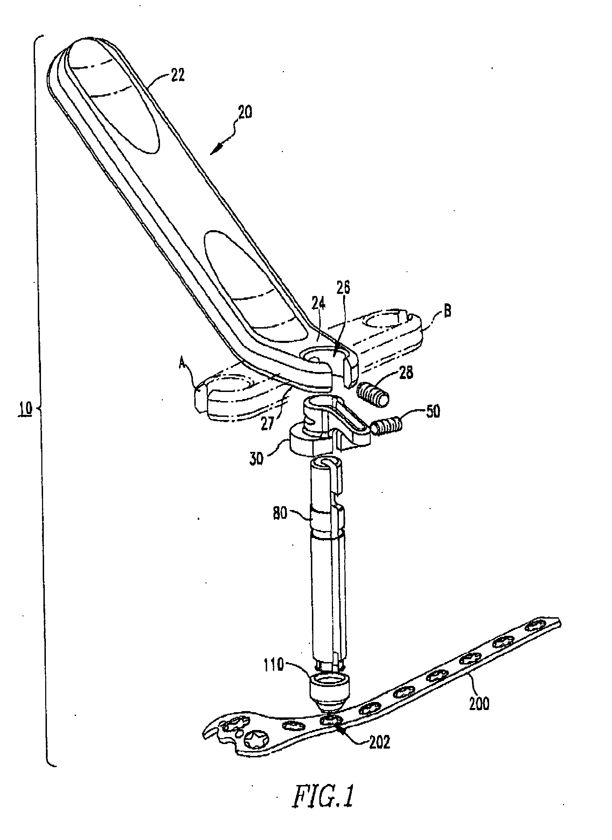

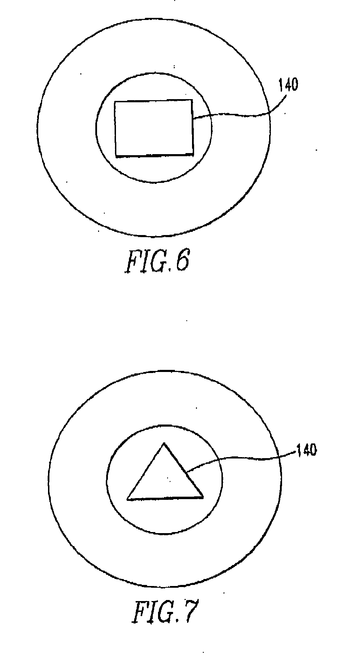

FIG. 1 is an exploded view of a drill guide assembly 10. In the depicted embodiment, the drill guide assembly 10 includes a guide collar 30, a guide sleeve 80, and a drill guide tip 110. The drill guide assembly 10 may include a drill guide handle 20. The drill guide handle 20 may be removably attached to a portion of the drill guide assembly 10. The drill guide assembly 10 is adapted for removable connection to a bone plate 200 and for guiding a drill bit 300 (best seen in FIG. 6) through a hole 202 in the bone plate 200. As is explained in greater detail below, after the drill guide handle 20 connects to a portion of the drill guide assembly 10 and the drill guide assembly 10 is resting on the bone plate 200, the drill guide handle 20 may be used to pivot the guide sleeve 80 to obtain a desired location of the drill bit 300. Phantom lines A and B illustrate exemplary rotational positions of the handle 20 but the handle 20 may be pivoted to other rotational positions as well.

The drill guide assembly 10 may be made from a metal, plastic, or composite. In the depicted embodiments, the drill guide assembly 10 is constructed from stainless steel and each piece of the assembly is a machined component. Alternatively, the drill guide assembly 10 could be an injection molded one-piece design.

The handle 20 includes a grip portion 22 and an engagement portion 24. In the depicted embodiment, grip portion 22 is transverse to the engagement portion 24. However, in some embodiments, the grip portion 22 is substantially planar with engagement portion 24. The engagement portion 24 includes an opening 26 and may have a generally planar lower surface 27. The opening 26 receives a portion of the guide collar 30, and, in some embodiments, the lower planar surface 27 engages the collar 30. In the depicted embodiment, the opening 26 is generally c-shaped. Optionally, the handle 20 includes a first ball plunger 28. The handle 20 may be made from any number of materials, including wood, metal, plastic, or a composite.

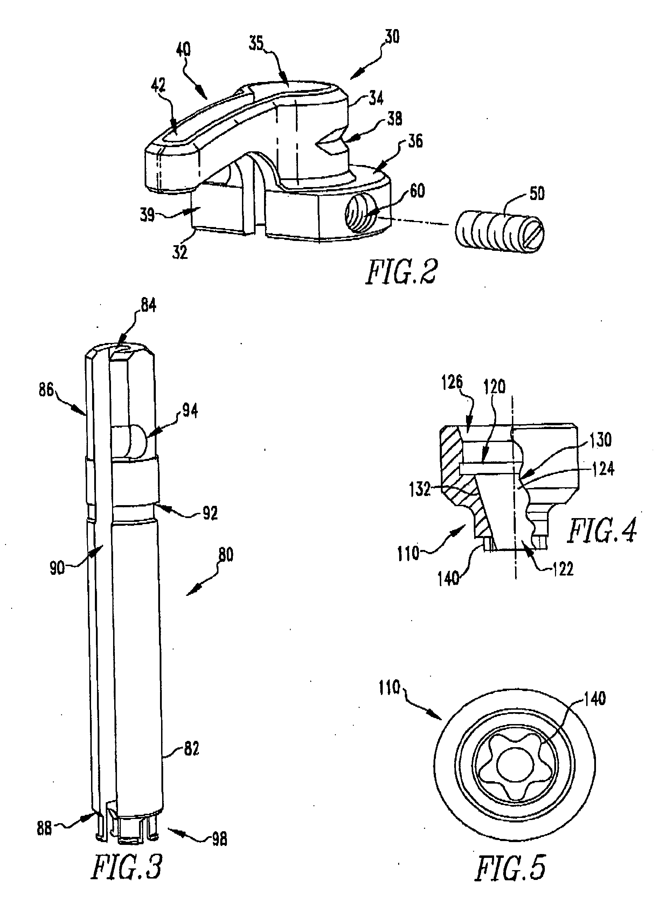

Referring now to FIG. 2, the guide collar 30 includes a base 32, a body portion 34, and an outrigger 40. The "outrigger" may alternatively be termed a "snout" or an "elongated feature." The body portion 34 extends from the base 32 in a transverse direction. In the depicted embodiment, the base 32 and the body portion 34 are generally cylindrical but other shapes may be used. The base 32 and the body portion 34 include a first bore 35. The body portion 34 is sized and dimensioned such that the base 32 provides a ledge 36. The ledge 36 provides a support for the handle 20. In some embodiments, the body portion 34 includes a first groove 38. The first groove 38 may be used in conjunction with the first ball plunger 28. In this manner, the handle 20 may be temporarily affixed to the collar 30. Optionally, the base 32 may include a substantially planar face 39. In the depicted embodiment, the outrigger 40 extends in an arcuate direction from the body portion 34, but the outrigger 40 may extend transversely from the body portion 34 in other embodiments.

The outrigger 40 includes an opening, a hole, or a slot 42. In some embodiments, the slot 42 is dimensioned for a single drill size. For example, the slot 42 may be dimensioned to receive a 2.7 millimeter drill bit. In other embodiments, the slot 42 is dimensioned to receive multiple sizes of drill bits. The slot 42 connects to the first bore 35. The slot 42 guides the drill bit 300. The length of the slot 42 is dimensioned according to the desired maximum angle of the drill. The maximum angle may be as much as thirty degrees, but in the depicted embodiment the maximum angle is fifteen degrees. In some embodiments, the outrigger 40 may include markings indicating an angle of the drill relative to the central axis of the guide sleeve 80. For example, these markings may include text or other markers indicating 2.5 degrees, 5.0 degrees, 7.5 degrees, 10 degrees, 12.5 degrees, and 15 degrees. Optionally, the guide collar 30 may include a second ball plunger 50 and a threaded hole 60 to receive the second ball plunger 50. The second ball plunger 50 may be located on either side of the guide collar 30.

Referring now to FIG. 3, the guide sleeve 80 includes sleeve body 82, a second bore 84, a proximal end portion 86, a distal end portion 88, a passage 90, and one or more tangs 98. A length of the guide sleeve 80 is determined by a length of the drill bit 300. In other words, the length of the guide sleeve 80 is designed according to the length of the drill bit 300. The sleeve body may be any shape, but the sleeve body 82 is generally cylindrical in FIG. 3. In the depicted embodiment, the second bore 84 is generally coaxial with the sleeve body 82. The second bore 84 extends substantially the entire length of the sleeve body 82 and is dimensioned to receive the drill bit 300. The proximal end portion 86 is dimensioned to fit within the first bore 35 of the collar 30. The tangs 98 extend generally from the distal end portion 88. In the depicted embodiment, there are four tangs, but those of ordinary skill in the art would understand that a greater or lesser number of tangs may be used. Optionally, the guide sleeve 80 may include a marker groove 92. The marker groove 92 may be painted or colored to indicate the drill guide assembly 10 is designed for use with a particular size or shape of drill. Alternatively, the marker groove 92 may be painted or colored to indicate the drill guide assembly 10 is part of a particular kit. In some embodiments, the guide sleeve 80 may include a u-shaped cutout 94. The u-shaped cutout 94 provides clearance for the second ball plunger 50.

Referring to FIGS. 4 and 5, the drill guide tip 110 includes an under-cut groove 120, a distal opening 122, a central axis 124, a proximal opening 126, a cone 130, and a locating boss 140. The under-cut groove 120 receives the tangs 98. The distal opening 122 opens up into the hole 202 when the drill guide tip 110 is engaged with the bone plate 200. The proximal opening 126 is sized and dimensioned to receive the distal end portion 88 of the guide sleeve 80. The cone 130 guides the drill bit 300. In other words, the cone 130 limits the drill bit 300 as the drill moves in the slot 42. The cone 130 has a cone wall 132 that is angled relative to the central axis 124. The maximum angle may be as much as thirty degrees, but in the depicted embodiment the maximum angle is fifteen degrees. In the depicted embodiments, the maximum angle of the cone wall 132 coincides with the maximum angle provided by the slot 42.

The locating boss 140 is adapted to engage the hole 202 of the bone plate 200. The locating boss 140 may be threaded such that it may thread into the hole 202. Alternatively, the locating boss 140 may have a geometric shape that corresponds with a portion of the hole 202. As examples, the locating boss 140 may have a star shape (best seen in FIG. 5), a square or rectangular shape (best seen in FIG. 6), or a triangular shape (best seen in FIG. 7). However, those having ordinary skill in the art would understand that other shapes may be used. What is significant is that the locating boss 140 engages the hole 202 of the bone plate 200 and prevents the drill guide tip 110 from rotating.

The drill guide assembly 10 is assembled in the following steps. First, the collar 30 is assembled to the proximal end portion 86 of the guide sleeve 80. As noted above, the proximal end portion 86 is dimensioned to fit within the first bore 35 of the collar 30. The fit between the two the components may be a slip fit or a press fit. In some embodiments, a structural adhesive is applied to the proximal end portion 86 before it is assembled to the collar 30. The guide sleeve 80 is inserted into the collar 30 such that the passage 90 is aligned with the slot 42. Optionally, once the collar 30 is attached to the guide sleeve 80, the ball plunger 50 may be attached to the collar such that the ball protrudes into the passage 90. In this manner, the second ball plunger 50 acts as indicator to let the user know whether the drill bit 300 is on-axis or off axis. In other words, the second ball plunger 50 acts as indicator to let the user know whether or not the drill bit 300 is aligned with the second bore 84. Second, the drill guide tip 110 is pressed onto the distal end portion 88 of the guide sleeve 80 until the tangs 98 engage the under-cut groove 120. Once the tangs 98 engage and capture the under-cut groove 120, the drill guide tip 110 can rotate 360 degrees about the longitudinal axis of the guide sleeve 80 but cannot easily be removed from the guide sleeve. Third, the handle 20 connects to the collar 30. The opening 26 is placed over the body portion 34, and the engagement portion 24 is pressed downwardly until it engages the ledge 36. Optionally, the handle 20 may include the first ball plunger 28, and the first ball plunger 28 may engage the first groove 38. Thereafter, the handle 20 may be used to pick up the drill guide assembly 10 and place it relative to the bone plate 200.

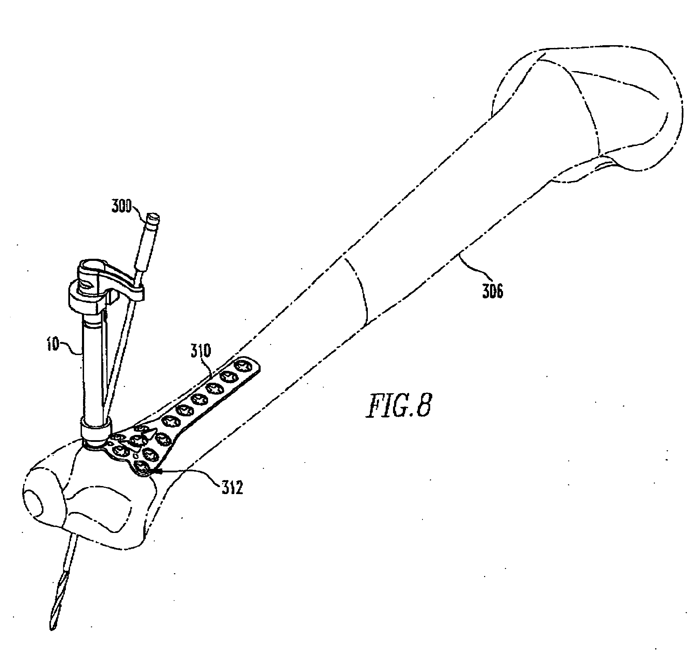

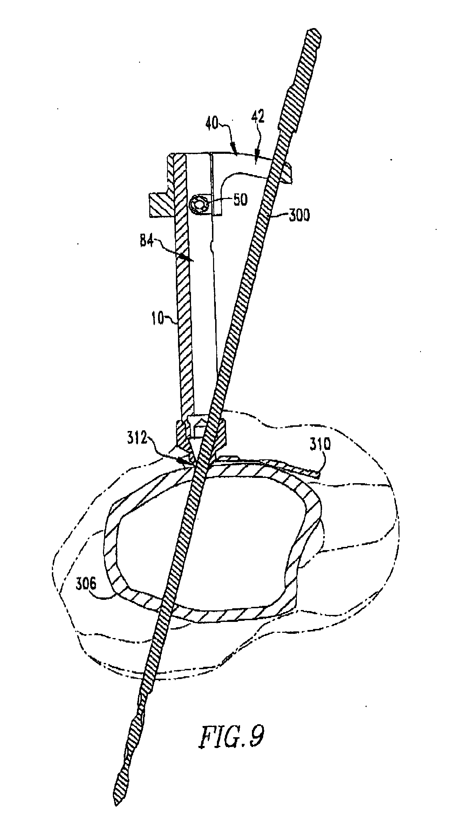

FIGS. 8 and 9 illustrate the drill guide assembly 10, the drill bit 300, a bone 306, and a bone plate 310. In the depicted embodiments, the bone 306 is a tibia, but those having ordinary skill in the art would understand that the invention is equally applicable to bone plating of other bones. The "drill bit" alternatively may be termed "twist drill." The bone plate 310 has a plurality of holes 312. In use, the drill guide assembly 10 is placed in contact with one of the holes 312. In some embodiments, the drill guide tip 110 rests on the bone plate 310. In other embodiments, the drill guide tip 110 protrudes through the hole 312 and rests on the bone 306. Optionally, the handle 20 may be used to place the drill guide assembly in contact with the hole 312 of the bone plate 310. Once the drill guide tip 110 is engaged with the bone plate 310, the user may rotate the collar 30. However, if the user is to drill on-axis, rotation may not be required. The user connects the drill bit 300 to a drill (not shown). The user inserts the drill bit 300 into the second bore 84. Optionally, the user may angle the drill bit 300 into the slot 42 and the cone 130. The outrigger 40 and the slot 42 provide support and alignment to a portion of the drill bit while drilling off-axis.

As best seen in FIG. 9, the drill bit may be placed on-axis (i.e., coaxial with the second bore 84), at the maximum angle at the end of the slot 42, or any place in between. In some embodiments, the second ball plunger 50 indicates the position from which the drill bit 300 moves from on-axis to off-axis. Thereafter, the user rotates the drill bit 300 using the drill to create a fastener hole in the bone 306. Optionally, the user may move the drill guide assembly 10 to another hole 312 and repeat the process.

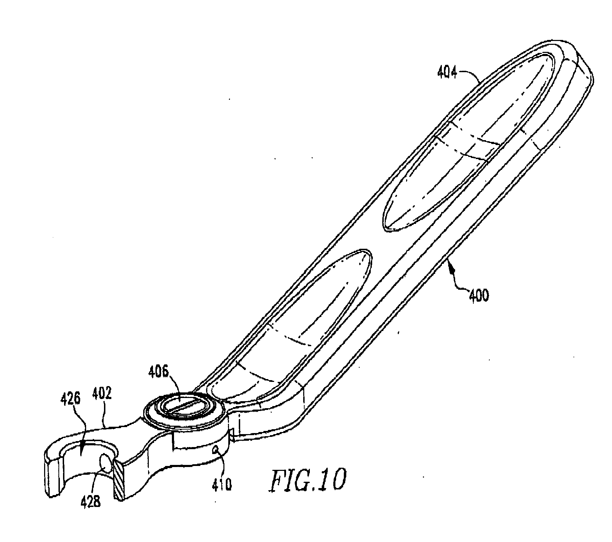

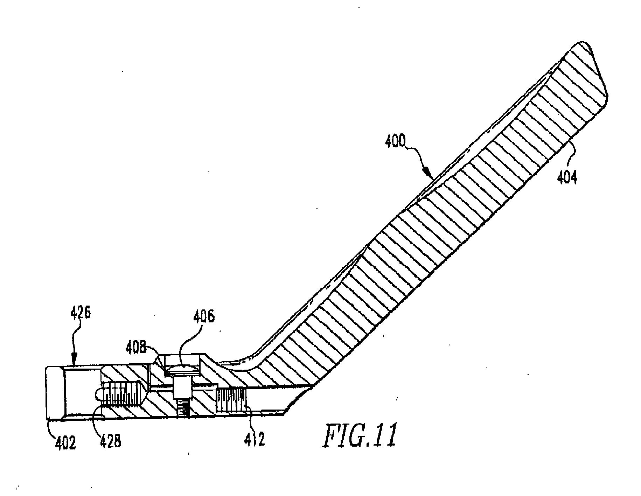

FIGS. 10 and 11 illustrate an alternative embodiment of the handle 400. The handle 400 includes a front pivot 402 and a grip portion 404. The pivoting handle 400 delivers enhanced accessibility and visibility through its multi-positional front pivot 402. The front portion 402 is connected to the grip portion 404 through the use of a fastener 406. In the depicted embodiment, the fastener 406 is a shoulder bolt. In some embodiments, the handle 400 also includes a disc spring or Belleville washer 408. The disc spring 408 places a biasing force on the fastener 406. The fastener 406 connects the grip portion 404 and the front pivot 402 in such a way that the front pivot 402 is pivotable relative to the grip portion. In some embodiments, the front pivot 402 is selectively positionable relative to the grip portion 404. In these embodiments, the front pivot and/or the grip portion has locking features that temporarily lock the front pivot in the desired location. For example, the front pivot 402 and/or the grip portion may have circumferentially spaced detents 410 and third ball plungers 412 that engage the detents 410. The handle 400 further includes an opening 426 to receive the collar 30 and may include a fourth ball plunger 428. The multi-positional rotating front pivot 402 rotates outside of the longitudinal axis of the drill guide itself for increased accessibility to the fracture site.

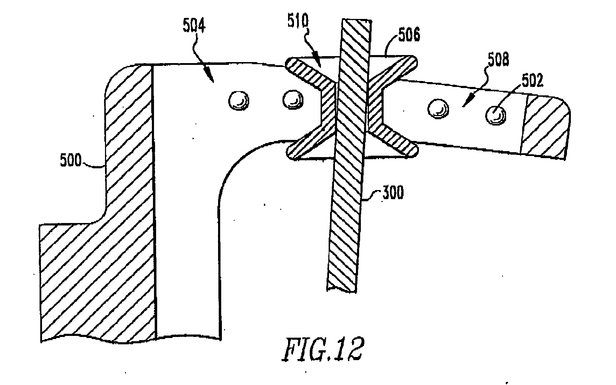

FIG. 12 illustrates an alternative embodiment of the outrigger 500. The outrigger 500 maintains alignment and supports the more proximal portion of the drill bit 300. The outrigger 500 may incorporate additional features that allow the user to positively identify off-axis drilling at a number of different angular degree increments. For example, the outrigger 500 may include detents or protrusions 502 located in a slot 504 and a flanged bobbin 506 that rides in the slot 504. The detents 502 are located such that spaces 508 positionally coincide with discrete angular position of the drill bit 300. The flanged bobbin 506 and the detents 502 are sized and dimensioned such that the flanged bobbin 506 can be pushed past the detents 502 but the detents 502 also temporarily hold the flanged bobbin 506 in one of the spaces 508. In some embodiments, the flanged bobbin 506 is constructed of a material, such as spring steel, that at least partially collapses when pressed against the detents 502 but returns to its normal size and shape when located in one of the spaces 508. The drill bit 300 may be inserted into an aperture 510 of the flanged bobbin 506, and the flanged bobbin 506 may be selectively positioned in the spaces 508 to angularly position the drill bit 300.

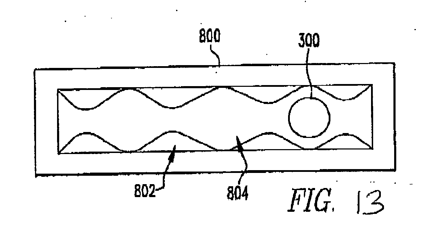

FIG. 13 illustrates yet another embodiment of the outrigger 800. The outrigger 800 includes a series of scallops 802 that form spaces 804. The spaces 804 positionally coincide with discrete angular position of the drill bit 300. The scallops 802 and the spaces 804 are dimensioned such that the drill bit 300 easily rotates in the spaces 804 but slightly interferes with the scallops 802. In this manner, the drill bit 300 can be pushed past the scallops 802 but the scallops 802 also temporarily hold the drill bit 300 in one of the spaces 804. The drill bit 300 may be selectively positioned in one of the spaces 804 to select the angular position of the drill bit 300.

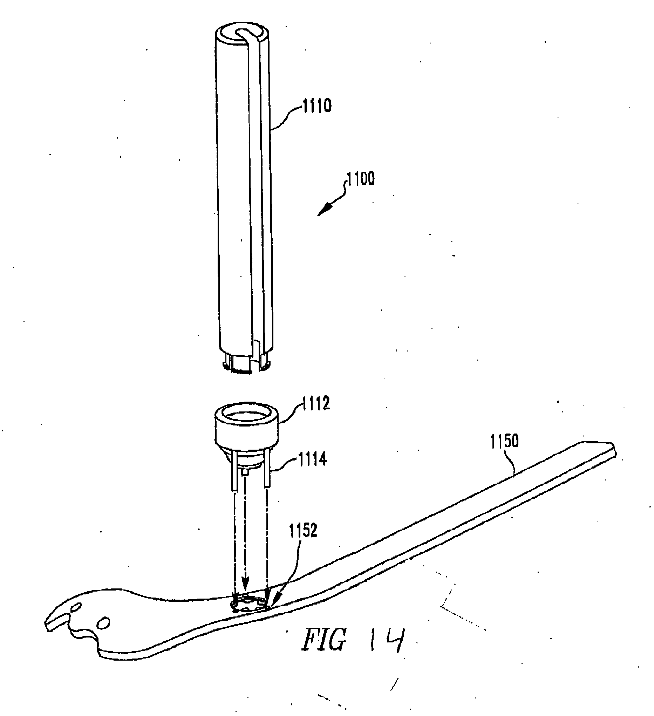

FIG. 14 illustrates an alternative embodiment of the drill guide assembly. FIG. 14 illustrates the drill guide assembly 1100. The drill guide assembly 1100 includes a drill sleeve 1110 and a drill guide tip 1112. In some embodiments, the drill guide assembly 1100 further includes a guide collar. The drill guide tip 1112 includes a plurality of locating members 1114. The locating members 1114 may be any number of shapes but are cylindrical in the depicted embodiment. In FIG. 14, three locating members 1114 are shown but any number of locating members may be used. The locating members 1114 correspond with and are adapted to engage locating holes 1152 located on bone plate 1150. In this embodiment, the guide sleeve 1110 is rotatable relative to the distal guide tip 1112. Unlike the embodiments depicted in FIGS. 4-7, the distal guide tip 1112 does not have a locating but instead utilizes the locating members 1114 to position the distal guide tip 1112 relative to the bone plate 1150. In some embodiments, the bone plate 1150 has sufficient thickness to allow the distal guide tip 1112 to pivot relative to the bone plate 1150.

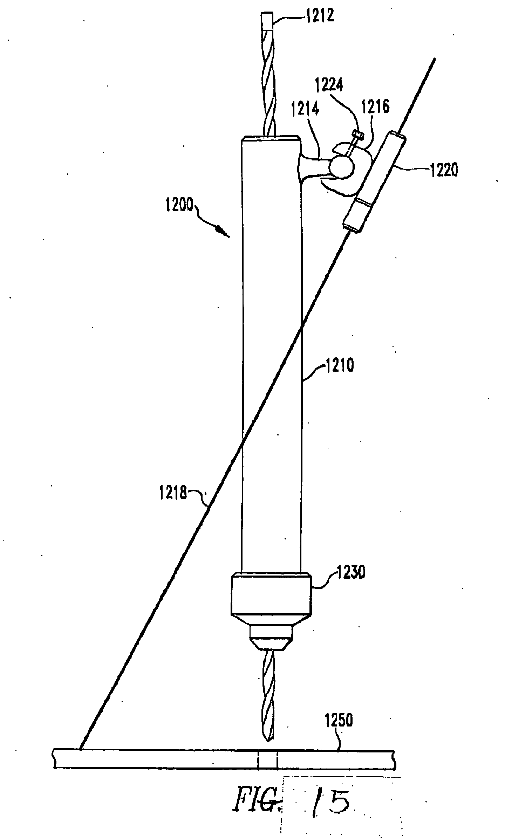

FIG. 15 is a front view of an alternative method of limiting the relationship between the bone plate and the drill guide assembly. FIG. 15 illustrates a drill guide assembly 1200 and a bone plate 1250. The drill guide assembly 1200 includes a drill sleeve 1210 and a drill guide tip 1230. The bone plate 1250 includes one or more holes (not shown). The drill guide assembly 1200 fits into the intended bone plate hole. The bone plate hole does not have a feature to hold the drill guide assembly 1200 at a particular angle or particular angular rotation relative to the hole. Therefore, the distal guide tip 1230 does not have a locating boss or locating member. The drill sleeve 1210 includes one or more spherical joints 1214. A wire holder 1220 operatively connects to the spherical joint 1214. In the depicted embodiment, a C-clamp and set screw 1224 are used to grip the spherical joint 1214. The wire holder 1220 is used to grip a wire 1218, such as a Kirschner wire, and apply a tensioning force to the drill guide assembly 1200 via the spherical joint 1214. As examples, the wire 1218 may be temporarily affixed to bone or the bone plate 1250. Several wire holders 1220 and wires 1218 may be necessary to adequately position and stabilize the drill guide assembly 1200. Thus, the surgeon may select a desired angular position and rotation for the drill guide assembly 1200 and use the anchored wires 1218 to temporarily affix the drill guide assembly in the chosen placement.

Also described herein is a method of drilling a hole into bone through a bone plate. The method includes the steps of engaging a guide tip of a variable angle drill guide assembly in contact with the bone plate, selecting a rotational position, inserting a drill bit into the variable angle drill guide assembly, selecting an angular position of the drill bit, and drilling the bone. Optional steps may include identifying the fracture type, selecting a bone plate, reducing the fracture, opening a surgical site, identifying an appropriate placement of the bone plate, temporarily affixing the bone plate into a desired position, and fastening the bone plate to the bone. The step of identifying the fracture type may include the step of x-raying the bone. The step of fracture reduction may be accomplished through manual reduction or through compression screws that engage the bone plate and provide axial compression. The step of temporarily affixing the bone plate may include the steps of clamping and/or attaching the bone plate via provisional fixation. The step of fastening the bone plate to the bone may be accomplished through the step of installing a locking screw and/or a non-locking screw.

In view of the foregoing, it will be seen that the several advantages of the invention are achieved and attained.

The embodiments were chosen and described in order to best explain the principles of the invention and its practical application to thereby enable others skilled in the art to best utilize the invention in various embodiments and with various modifications as are suited to the particular use contemplated.

As various modifications could be made in the constructions herein described and illustrated without departing from the scope of the invention, it is intended that all matter contained in the foregoing description or shown in the accompanying drawings shall be interpreted as illustrative rather than limiting. For example, while FIG. 1 illustrates a distal guide tip, other structure may be used to temporarily maintain contact between the guide sleeve and the bone plate. Thus, the breadth and scope of the present invention should not be limited by any of the above-described exemplary embodiments but should be defined only in accordance with the following claims appended hereto and their equivalents.

## Claims

1. A drill guide assembly (10;1100;1200) adapted for removable connection to a bone plate (200;1150;1250) and for guiding a drill bit (300) through at least one hole (202) of the bone plate, the at least one hole having a central axis, the drill guide assembly comprising:
a. a guide sleeve (80;1110) having a proximal end portion (86) and a distal end portion (88), a sleeve body (82), a central axis and a length, the guide sleeve also having a bore (84) coaxial with the sleeve body and a passage (90) wherein the passage (90) extend at least a portion of the length of the guide sleeve, the guide sleeve being coaxial with the central axis of the at least one hole and adapted to swivel about the central axis of the at least one hole while the distal end portion maintains contact with the bone plate; and
b. a guide collar (30) operatively connected to the proximal end portion, the guide collar having an outrigger (40), the outrigger having a slot (42) which is adapted to receive the drill bit and which is aligned with the passage (90), the slot (42) allowing an operator to select an angle for placement of the drill bit with respect to the central axis of the guide sleeve, and wherein the length of the slot (42) is dimensioned according to the desired maximum angle of the drill.

2. The drill guide assembly according to claim 1, wherein the outrigger further comprises a flanged bobbin (506).

3. The drill guide assembly according to claim 1, wherein the outrigger further comprises a plurality of scallops.

4. The drill guide assembly according to any of claims 1-3, further comprising at least one ball plunger (28;50).

5. The drill guide assembly according to any of claims 1-4, wherein the guide collar includes a base (32) and a body portion (34), and the outrigger extends generally from the body portion.

6. The drill guide assembly according to any of claims 1-5, wherein the guide sleeve further comprises at least one marker groove.

7. The drill guide assembly according to any of claims 1-6, wherein the outrigger includes one or more markings to indicate an angle of the drill bit.

8. The drill guide assembly according to any of claims 1-7, further comprising a drill guide handle (20), the drill guide handle having a grip portion (22) and an engagement portion (24).

9. The drill guide assembly of claim 8, wherein the drill guide handle further comprises a pivot portion.

10. The drill guide assembly according to any of claims 1-9, wherein the distal end portion of the guide sleeve comprises a spherical tip.

11. The drill guide assembly according to claim 10, further comprising a bushing and a distal guide tip (110).

12. The drill guide assembly according to claim 11, further comprising a limiter (130).

13. The drill guide assembly according to claim 12, wherein the limiter (130) threadingly engages the guide sleeve.

14. The drill guide assembly according to any of claims 1-9, wherein the distal end portion of the guide sleeve comprises a drill guide tip (110).

15. The drill guide assembly according to claim 14, wherein the drill guide tip comprises an anti-rotation feature (140).

16. The drill guide assembly according to claim 15, wherein the anti-rotation feature (140) is a locating boss (140).

17. The drill guide assembly according to claim 15, wherein the anti-rotation feature (140) comprises at least one thread adapted to mate with at least one thread of the at least one bone plate hole (202).

18. The drill guide assembly of claim 15, wherein the distal guide tip (110) includes a locating member (140).

19. The drill guide assembly according to any of claims 14-18, wherein the guide sleeve further comprises at least one tang (98) and the drill guide tip further comprises an under-cut groove (120) adapted to receive the at least one tang.

20. The drill guide assembly according to any of claims 14-19, wherein the drill guide tip further comprises a cone.

## Patentansprüche

1. Eine Bohrführungsanordnung (10; 1100; 1200), die angepasst ist für eine lösbare Verbindung mit einer Knochenplatte (200; 1150; 1250) und zum Führen eines Bohreinsatzes (300) durch mindestens ein Loch (202) der Knochenplatte, wobei das mindestens eine Loch eine Mittelachse aufweist, wobei die Bohrführungsanordnung Folgendes beinhaltet:
a. eine Führungshülse (80; 1110), die einen proximalen Endabschnitt (86) und einen distalen Endabschnitt (88), einen Hülsenkörper (82), eine Mittelachse und eine Länge aufweist, wobei die Führungshülse auch eine Bohrung (84), die mit dem Hülsenkörper koaxial ist, und einen Durchlass (90) aufweist, wobei sich der Durchlass (90) mindestens über einen Abschnitt der Länge der Führungshülse erstreckt, wobei die Führungshülse mit der Mittelachse des mindestens einen Lochs koaxial ist und angepasst ist, um sich um die Mittelachse des mindestens einen Lochs zu drehen, während der distale Endabschnitt den Kontakt mit der Knochenplatte wahrt; und
b. einen Führungskragen (30), der betriebsfähig mit dem proximalen Endabschnitt verbunden ist, wobei der Führungskragen einen Ausleger (40) aufweist, wobei der Ausleger einen Schlitz (42) aufweist, der angepasst ist, um den Bohreinsatz aufzunehmen, und der auf den Durchlass (90) ausgerichtet ist, wobei der Schlitz (42) einem Benutzer ermöglicht, einen Winkel für die Platzierung des Bohreinsatzes in Bezug auf die Mittelachse der Führungshülse auszuwählen, und wobei die Länge des Schlitzes (42) gemäß dem gewünschten maximalen Winkel des Bohrers bemessen ist.

2. Bohrführungsanordnung gemäß Anspruch 1, wobei der Ausleger ferner eine angeflanschte Trommel (506) beinhaltet.

3. Bohrführungsanordnung gemäß Anspruch 1, wobei der Ausleger ferner eine Vielzahl von Wellungen beinhaltet.

4. Bohrführungsanordnung gemäß einem der Ansprüche 1-3, die ferner mindestens ein Kugeldruckstück (28; 50) beinhaltet.

5. Bohrführungsanordnung gemäß einem der Ansprüche 1-4, wobei der Führungskragen eine Basis (32) und einen Körperabschnitt (34) umfasst und wobei sich der Ausleger im Allgemeinen von dem Körperabschnitt erstreckt.

6. Bohrführungsanordnung gemäß einem der Ansprüche 1-5, wobei die Führungshülse ferner mindestens eine Markierungsrille beinhaltet.

7. Bohrführungsanordnung gemäß einem der Ansprüche 1-6, wobei der Ausleger eine oder mehrere Markierungen umfasst, um einen Winkel des Bohreinsatzes anzuzeigen.

8. Bohrführungsanordnung gemäß einem der Ansprüche 1-7, die ferner einen Bohrführungshaltegriff (20) beinhaltet, wobei der Bohrführungshaltegriff einen Griffabschnitt (22) und einen Eingriffsabschnitt (24) aufweist.

9. Bohrführungsanordnung gemäß Anspruch 8, wobei der Bohrführungshaltegriff ferner einen Schwenkabschnitt beinhaltet.

10. Bohrführungsanordnung gemäß einem der Ansprüche 1-9, wobei der distale Endabschnitt der Führungshülse eine kugelförmige Spitze beinhaltet.

11. Bohrführungsanordnung gemäß Anspruch 10, die ferner eine Buchse und eine distale Führungsspitze (110) beinhaltet.

12. Bohrführungsanordnung gemäß Anspruch 11, die ferner einen Begrenzer (130) beinhaltet.

13. Bohrführungsanordnung gemäß Anspruch 12, wobei der Begrenzer (130) gewindeartig in die Führungshülse eingreift.

14. Bohrführungsanordnung gemäß einem der Ansprüche 1-9, wobei der distale Endabschnitt der Führungshülse eine Bohrführungsspitze (110) beinhaltet.

15. Bohrführungsanordnung gemäß Anspruch 14, wobei die Bohrführungsspitze ein Antirotationsmerkmal (140) beinhaltet.

16. Bohrführungsanordnung gemäß Anspruch 15, wobei das Antirotationsmerkmal (140) ein Fixierungsansatz (140) ist.

17. Bohrführungsanordnung gemäß Anspruch 15, wobei das Antirotationsmerkmal (140) mindestens ein Gewinde beinhaltet, das angepasst ist, um mit mindestens einem Gewinde des mindestens einen Knochenplattenlochs (202) zusammenzupassen.

18. Bohrführungsanordnung gemäß Anspruch 15, wobei die distale Führungsspitze (110) ein Fixierungselement (140) umfasst.

19. Bohrführungsanordnung gemäß einem der Ansprüche 14-18, wobei die Führungshülse ferner mindestens einen Dorn (98) beinhaltet und die Bohrführungsspitze ferner eine hinterschnittene Rille (120) beinhaltet, die angepasst ist, um den mindestens einen Dorn aufzunehmen.

20. Bohrführungsanordnung gemäß einem der Ansprüche 14-19, wobei die Bohrführungsspitze ferner einen Trichter beinhaltet.

## Revendications

1. Un ensemble guide-foret (10 ; 1100 ; 1200) adapté pour être raccordé de façon amovible à une plaque d'ostéosynthèse (200 ; 1150 ; 1250) et pour guider une mèche (300) à travers au moins un trou (202) de la plaque d'ostéosynthèse, cet au moins un trou ayant un axe central, l'ensemble guide-foret comprenant :
a. un manchon de guidage (80 ; 1110) ayant une portion d'extrémité proximale (86) et une portion d'extrémité distale (88), un corps de manchon (82), un axe central et une longueur, le manchon de guidage ayant aussi un alésage (84) coaxial avec le corps de manchon et un passage (90), le passage (90) s'étendant sur au moins une portion de la longueur du manchon de guidage, le manchon de guidage étant coaxial avec l'axe central de cet au moins un trou et adapté pour pivoter autour de l'axe central de cet au moins un trou tandis que la portion d'extrémité distale maintient le contact avec la plaque d'ostéosynthèse ; et
b. un collier de guidage (30) raccordé de façon opérationnelle à la portion d'extrémité proximale, le collier de guidage ayant un support en saillie (40), le support en saillie ayant une fente (42) qui est adaptée pour recevoir la mèche et qui est alignée avec le passage (90), la fente (42) permettant à un opérateur de choisir un angle de mise en place de la mèche par rapport à l'axe central du manchon de guidage, et dans lequel la longueur de la fente (42) est dimensionnée selon l'angle maximal souhaité du foret.

2. L'ensemble guide-foret selon la revendication 1, dans lequel le support en saillie comprend en sus une bobine à rebords (506).

3. L'ensemble guide-foret selon la revendication 1, dans lequel le support en saillie comprend en sus une pluralité de festons.

4. L'ensemble guide-foret selon n'importe lesquelles des revendications 1 à 3, comprenant en sus au moins un poussoir à bille (28 ; 50).

5. L'ensemble guide-foret selon n'importe lesquelles des revendications 1 à 4, dans lequel le collier de guidage inclut une base (32) et une portion de corps (34), et le support en saillie s'étend généralement à partir de la portion de corps.

6. L'ensemble guide-foret selon n'importe lesquelles des revendications 1 à 5, dans lequel le manchon de guidage comprend en sus au moins une rainure de marquage.

7. L'ensemble guide-foret selon n'importe lesquelles des revendications 1 à 6, dans lequel le support en saillie inclut une ou plusieurs marques pour indiquer un angle de la mèche.

8. L'ensemble guide-foret selon n'importe lesquelles des revendications 1 à 7, comprenant en sus une poignée de guide-foret (20), la poignée de guide-foret ayant une portion de préhension (22) et une portion de mise en prise (24).

9. L'ensemble guide-foret de la revendication 8, dans lequel la poignée de guide-foret comprend en sus une portion formant pivot.

10. L'ensemble guide-foret selon n'importe lesquelles des revendications 1 à 9, dans lequel la portion d'extrémité distale du manchon de guidage comprend un embout sphérique.

11. L'ensemble guide-foret selon la revendication 10, comprenant en sus une douille et un embout de guidage distal (110).

12. L'ensemble guide-foret selon la revendication 11, comprenant en sus un limiteur (130).

13. L'ensemble guide-foret selon la revendication 12, dans lequel le limiteur (130) se met en prise par vissage avec le manchon de guidage.

14. L'ensemble guide-foret selon n'importe lesquelles des revendications 1 à 9, dans lequel la portion d'extrémité distale du manchon de guidage comprend un embout de guide-foret (110).

15. L'ensemble guide-foret selon la revendication 14, dans lequel l'embout de guide-foret comprend un élément antirotation (140).

16. L'ensemble guide-foret selon la revendication 15, dans lequel l'élément antirotation (140) est un bossage de positionnement (140).

17. L'ensemble guide-foret selon la revendication 15, dans lequel l'élément antirotation (140) comprend au moins un filetage adapté pour s'engrener avec au moins un taraudage de cet au moins un trou de plaque d'ostéosynthèse (202).

18. L'ensemble guide-foret selon la revendication 15, dans lequel l'embout de guidage distal (110) inclut un organe de positionnement (140).

19. L'ensemble guide-foret selon n'importe lesquelles des revendications 14 à 18, dans lequel le manchon de guidage comprend en sus au moins un tenon (98) et l'embout de guide-foret comprend en sus une rainure de dégagement (120) adaptée pour recevoir cet au moins un tenon.

20. L'ensemble guide-foret selon n'importe lesquelles des revendications 14 à 19, dans lequel l'embout de guide-foret comprend en sus un cône.
